# EUROPÄISCHE PATENTSCHRIFT

(11) **EP 0 661 983 B1**
(45) Veröffentlichungstag und Bekanntmachung des Hinweises auf die Patenterteilung: **01.05.1996**
(21) Anmeldenummer: 93919311.6
(22) Anmeldetag: 09.09.1993
(51) Int. Cl.: A61K 35/78

(54) **VERWENDUNG VON BÄRLAUCH ZUR THERAPIE ODER PROPHYLAXE VON DURCHBLUTUNGSSTÖRUNGEN**
USE OF WILD GARLIC FOR THE THERAPY OR PREVENTION OF BLOOD CIRCULATION DISTURBANCES
UTILISATION D'AIL DES OURS DANS LE TRAITEMENT OU LA PROPHYLAXIE DES TROUBLES CIRCULATOIRES

(30) Priorität: 09.09.1992 DE 4230188
(43) Veröffentlichungstag der Anmeldung: 12.07.1995
(73) Patentinhaber: PANDALIS, Georgios, D-49219 Glandorf (DE)
(72) Erfinder: PANDALIS, Georgios, D-49219 Glandorf (DE); KIESEWETTER, Holger, D-66424 Homburg (DE)
(74) Vertreter: Grünecker, Kinkeldey, Stockmair & Schwanhäusser Anwaltssozietät
(86) Internationale Anmeldenummer: EP9302442
(87) Internationale Veröffentlichungsnummer: WO9405306

(56) Entgegenhaltungen:
- BIOLOGICAL ABSTRACTS, xol. 95, no. 12 15. Juni 1993, Philadelphia, PA, US; abstract no. 134728, RIETZ B. ET AL. 'CARDIOPROTECTIVE ACTIONS OF WILD GARLIC (ALLIUM URSINUM) IN ISCHEMIA AND REPERFUSION'
- THE AMERICAN JOURNAL OF CHINESE MEDICINE Bd. 7, Nr. 3 , 1979 Seiten 197 - 236 V. PETKOV 'PLANTS WITH HYPOTENSIVE, ANTIATHEROMATOUS AND CORONARODILATATING ACTION'
- PLANTA MEDICA Bd. 58, Nr. 1 , Februar 1992 Seiten 1 - 7 A. SENDL ET AL. 'COMPARATIVE PHARMACOLOGICAL INVESTIGATIONS OF ALLIUM URSINUM AND ALLIUM SATIVUM'
- THERAPIEWOCHE Bd. 40, Nr. 46 , 12. November 1990 Seite 3349 HANS-JÜRGEN RICHTER 'PFLÜCKEN SIE SICH EINEN ACE-HEMMER !'
- BIOLOGICAL ABSTRACTS, vol. 95, no. 12 15. Juni 1993, Philadelphia, PA, US; abstract no. 134728, RIETZ B. ET AL. 'CARDIOPROTECTIVE ACTIONS OF WILD GARLIC (ALLIUM URSINUM) IN ISCHEMIA AND REPERFUSION'

## Beschreibung

Die Erfindung betrifft die Verwendung von Allium ursinum L. (Bärlauch).

Allium ursinum L. (Bärlauch, Bärenlauch, wilder Knoblauch) gehört, wie der Knoblauch, zur Familie der Liliaceae. Die Pflanze ist u.a. in fast ganz Europa beheimatet und wird auch als Heil-, Gewürz- und Gemüsepflanze kultiviert. Für Arzneimittel werden die Bärlauchzwiebel (Allii ursini bulbus), das Bärlauchkraut (Allii ursini herba) sowie die ganze, frische zur Blütezeit gesammelte Pflanze für homöopathische Zwecke verwendet. Die Inhaltsstoffe der Zwiebel und des Krautes sind als identisch anzusehen. (s. Hagers Handbuch der Pharmazeutischen Praxis, 4. Band, 5. Auflage, Springer-Verlag, im Druck).

In der Bärlauchzwiebel lassen sich abhängig von Extraktionsmedium und -verfahren unterschiedliche Muster an genuinen und nicht genuin enthaltenen Inhaltsstoffen nachweisen. Dazu gehören Cysteinsulfoxide, wie S-Methyl-L-(+)-cysteinsulfoxid und S-Allyl-L-(+)-cysteinsulfoxid (Alliin) und Thiosulfinate, die durch Fermentation aus den Cysteinsulfoxiden gebildet werden. Beispiele für die Thiosulfinate sind Allylmethylthiosulfinat bzw. Methylallylthiosulfinat, Diallylthiosulfinat (Allicin) und Dimethylthiosulfinat. Weitere, nicht genuin in der Bärlauchzwiebel enthaltene Stoffe sind Dithiine, wie Vinyldithiine, Ajoen (4,5,9-Trithiododeca-1,6,11-trien-9-oxid), das durch Selbstkondensation von Allicin entsteht, sowie Ajoenhomologe. Ferner werden als wasserdampfflüchtige Bestandteile (12 % bezogen auf die frische Bärlauchzwiebel) Methylallyltrisulfid, das den Hauptbestandteil des Wasserdampfdestillats bildet, sowie geringe Mengen an Diallyldisulfid und Diallyltrisulfid erhalten.

Diese Stoffe sind ebenfalls nicht genuin in der Bärlauchzwiebel enthalten. Weitere, genuin in der Bärlauchzwiebel enthaltene Stoffe sind beispielsweise die freien Aminosäuren L-Arginin, L-Asparaginsäure, L-Glutaminsäure, L-Asparagin, L-Glutamin, L-Glycin, L-Threonin und L-Alanin.

Die frische Zwiebel enthält nach der Fermentation etwa 0,05 bis 0,12 % Allicin.

Untersuchte Wirkungen der Bärlauchzwiebel sind eine Lipoxygenase- und Cyclooxygenasehemmung, sowie eine Hemmung im PAF-Thrombozytenaggregationstest.

Frische Bärlauchblätter enthalten nach der Fermentation etwa 0,005 % Allicin, getrocknete etwa 0,07 %. Ferner enthalten frische Bärlauchblätter etwa 0,007 % wasserdampfflüchtige Bestandteile, die den wasserdampfflüchtigen Bestandteilen der Bärlauchzwiebel entsprechen.

Volkstümlich werden Bärlauchblätter und -zwiebeln, ähnlich wie Allium sativum (Knoblauch), bei Magen-Darm-Störungen, Gärungsdysepsien, Flatuleszenz, gegen Bluthochdruck und Arteriosklerose eingesetzt. Äußerlich finden sie Anwendung bei Hautausschlägen. Die Wirksamkeit bei den genannten Anwendungsgebieten ist nicht belegt.

Die Urtinktur aus ganzen, frischen, zu Beginn der Blütezeit gesammelten Bärlauchpflanzen HAB1 ist eine goldgelbe Flüssigkeit mit Geruch und Geschmack nach Knoblauch HAB1. Die Anwendungsgebiete entsprechen dem homöopathischen Arzneimittelbild. Dazu gehört beispielsweise Verdauungsschwäche.

Ein Einsatz von Bärlauch in der Schulmedizin erfolgt nicht.

Bei Durchblutungsstörungen handelt es sich um organisch oder funktionell bedingte Minderdurchblutung eines Gewebes, die Ischämie zur Folge haben kann. Die Prüfung auf Durchblutungsstörungen erfolgt beispielsweise durch eine Lagerungsprobe, Druckmessungen, Belastungsmessungen oder, falls es ich um eine Mikrozirkulationsstörung handelt, mit Hilfe der Kapillarmikroskopie oder Sauerstoffdruckmessungen (Pschyrembel, Klinisches Wörterbuch, 255. Auflage, Verlag Walter de Gruyter, Berlin, New York 1986).

Durchblutungsstörungen werden bisher überwiegend mit chemischen Arzneimitteln behandelt.

Aufgabe der vorliegenden Erfindung ist es, ein weiteres Mittel zur Behandlung von Durchblutungsstörungen zur Verfügung zu stellen.

Diese Aufgabe wird erfindungsgemäß gelöst durch die Verwendung von Allium ursinum L. (Bärlauch) zur Herstellung eines Medikaments zur Behandlung oder Prophylaxe von Durchblutungsstörungen.

Zur Erzielung der durchblutungsfördernden Wirkung von Bärlauch kann die ganze Pflanze in frischem oder getrocknetem Zustand, sowie einzelne frische oder getrocknete Pflanzenteile, wie die Bärlauchzwiebel oder das Bärlauchkraut, eingesetzt werden. Weiterhin sind auch verschiedene Extrakte der Bärlauchzwiebel und/oder des -krauts, die durch Wasserdampfdestillation erhaltenen Fraktionen und die Urtinktur sowie Mischungen daraus wirksam.

Erfindungsgemäß wird Bärlauch bevorzugt in Form üblicher pharmazeutischer Zubereitungen, wie Lösungen, Konzentraten, Tabletten oder Kapseln, oral verabreicht. Besonders bevorzugt ist die Verabreichung von getrockneten, pulverisierten Bärlauchblättern. Das Pulver wird mit Wasser eingenommen. Die benötigte Dosierung der Wirkstoffe wird in Abhängigkeit von üblichen Faktoren, wie der Natur und Schwere der Erkrankung und dem Körpergewicht des Patienten, vom Arzt festgelegt.

Die Verwendung von Bärlauch bewirkt insbesondere eine Erweiterung der Widerstandsgefäße und ein Verflüssigen des Blutes.

Bärlauch verbessert bereits bei einer Tagesdosis von einem Gramm, als Einmaldosis verabreicht, die Mikrozirkulation der Haut. Die Zunahme der Erythrozytenruhegeschwindigkeit beträgt über 30 % und ist demnach klinisch relevant. Bei akralen Durchblutungsstörungen mit Raynaud-Phänomenen führt eine Steigerung des Runeflusses um fast 0,2 mm/s zum Abklingen der Beschwerden. Parallel zu einer Blutflußzunahme in der Peripherie nimmt die Plasmaviskosität ab. Somit scheint es durch Bärlauch zu einer endogenen Hämodilution zu kommen, indem Wasser aus dem Interstitium in die Gefäße eindringt und das Plasmavolumen vergrößert. Bärlauch scheint demnach eine vasoaktive Substanz zu sein.

Das folgende Beispiel zeigt die durchblutungsfördernde Wirkung von Bärlauch bei gesunden Probanden.

### Beispiel 1

Es wurde eine offene Phase I-Studie an 8 gesunden Probanden mit einer Plasmaviskosität größer 1,30 mPas durchgeführt, um die Dosisabhängigkeit der Wirkung von Bärlauchblättern auf die Erythrocytenruhegeschwindigkeit im Akutversuch zu ermitteln. Die konfirmatonische Zielgröße Erythrocytenruhegeschwindigkeit wurde durch Nagelfalzkapillaroskopie ermittelt.

### Probanden

Die Studie wurde an 8 gesunden Probanden beiderlei Geschlechts mit einem mittleren Alter von 26 + 4 Jahren, einer mittleren Körpergröße von 175 + 9 cm und einem mittleren Körpergewicht von 69 + 6 kg durchgeführt. 5 der Probanden waren Männer, 3 waren Frauen. Außer der Einnahme von Ovulationshemmern bei den 3 Frauen wurden während der Studie keine Medikamente eingenommen. Keiner der Probanden war Raucher. Diabetes mellitus, arterielle Hypertonie oder Fettstoffwechselstörungen lagen bei keinem der Probanden vor. Auch andere Erkrankungen konnten bei einer internistischen Untersuchung nicht festgestellt werden.

Die gesamte Prüfung gliederte sich in 3 Phasen, in denen jeweils folgenden Dosierungen von Bärlauchblättern einmalig appliziert wurden:

| | |
|---|---|
| Prüfphase 1: | 7,0 g |
| Prüfphase 2: | 3,5 g |
| prüfphase 3: | 1,0 g |

Die Dauer der Auswaschphase zwischen den einzelnen Prüfphasen betrug mindestens 14 Tage. In jeder Prüfphase wurden vor der Einnahme des Präparates sowie 5 Stunden danach folgende Prüfparameter bestimmt:

### Konfirmatorische Zielgröße:

- Erythrozytenruhegeschwindigkeit
   (Nagelfalzkapillaroskopie)

### Explorative Parameter

- Hämatokrit
- Plasmaviskosität
- Erythrozytenaggregation
- Thrombozytenaggregationsindex
- Gewebs-Plasminogen-Aktivator-Konzentration
- Blutdruck (systolisch/diastolisch)
- Herzfrequenz
Dazu wurden den Probanden jeweils 30 ml Blut entnommen. Ebenfalls vor der Einnahme des Prüfpräparates und 5 h danach erfolgte die Messung des arteriellen Blutdrucks und der Herzfrequenz.

### Prüfpräparat

Das Bärlauch-Präparat wurde in Form getrockneter, gemahlener Blätter appliziert. Die Probanden nahmen

| | |
|---|---|
| in Phase 1: | 7,0 g |
| in Phase 2: | 3,5 g |
| in Phase 3: | 1,0 g |

gelöst in 100 ml Wasser, unter Aufsicht des Prüfarztes ein.

Nach Prüfphase 1 und 2 erfolgte ein Vergleich der über alle 8 Probanden gemittelten konfirmatorischen Zielgröße vor und nach Gabe des Prüfpräparates. Eine Zunahme der Erythrozytenruhegeschwindigkeit von 20 % und mehr wurde als signifikant angesehen und führte zur Herabsetzung der Dosierung.

Es wurden folgene Ergebnisse erhalten:

### 1) Effekte von Bärlauch auf die Mikrozirkulation:

Die konfirmatorische Zielgröße war die Ruhegeschwindigkeit der Erythrozyten in den Nagelfalzkapillaren. Die Tabelle 1 zeigt die Ergebnisse der 3 Phasen.

**Tabelle 1**

| Erythrozytengeschwindigkeit vor und nach Bärlaucheinnahme | | | | | |
|---|---|---|---|---|---|
| | vorher | nach 5 h | Unterschied | % | P |
| 7 g Bärlauch | 0,46 ± 0,19 | 0,57 ± 0,13 | 0,12 ± 0,13 | 26 | 0,04 |
| 3,5 g " | 0,41 ± 0,22 | 0,56 ± 0,23 | 0,15 ± 0,11 | 37 | 0,006 |
| 1 g " | 0,46 ± 0,13 | 0,64 ± 0,09 | 0,19 ± 0,15 | 32 | 0,01 |

Die Zunahme der Erythrozytengeschwindigkeiten sind vergleichbar. Sie unterscheiden sich im Gruppenvergleich nicht signifikant. In der Prüfphase I nahm die Geschwindigkeit bei 6 Probanden relevant zu, in der Prüfphase II ebenfalls bei 6 Probanden und in der Prüfphase 3 auch bei 6 Probanden, wobei bei einer Person der Anstieg nur 15 % betrug. Die Responderrate betrug für alle 3 Dosierungen demnach 75 %.

### 2) Effekte von Bärlauch auf die Plasmaviskosität:

In der Tabelle 2 sind die Plasmaviskositäten vor und nach Bärlaucheinnahme zusammengestellt.

**Tabelle 2**

| Plasmaviskosität vor und nach Bärlaucheinnahme | | | | | |
|---|---|---|---|---|---|
| | vorher | nach 5 h | Unterschied | % | P |
| 7 g Bärlauch | 1,32 ± 0,04 | 1,29 ± 0,05 | 0,03 | 2,3 | 0,009 |
| 3,5 g " | 1,34 ± 0,03 | 1,30 ± 0,04 | 0,04 | 3,0 | 0,04 |
| 1 g " | 1,33 ± 0,03 | 1,30 ± 0,05 | 0,03 | 2,3 | 0,02 |

In allen 3 Prüfphasen, für die Einmaldosen von 7 g, 3,5 g und 1 g, sanken die Plasmaviskositäten relevant ab. Die Senkungen schwankten zwischen 2,3 und 3 %. Im Gruppenvergleich ergaben sich keine signifikanten Unterschiede zwischen den Abnahmen durch die verschiednen Dosierungen.

Die Erytrhozytengeschwindigkeit nahm in der Prüfphase 1 (Einnahme von 7 g Bärlauch) von 0,46 mm/s um 0,12 mm/s (26 %; p < 0,05) 5 Stunden nach der Einnahme, in Prüfphase 2 (Einnahme von 3,5 g Bärlauch) von 0,41 mm/s um 0,15 mm/s (37 %; p < 0,01) und in Prüfphase 3 von 0,46 mm/s um 0,19 mm/s (32 %; p = 0,01) zu. Die Plasmaviskositäten nahmen in allen 3 Prüfphasen in vergleichbarem Ausmaß relevant ab. Die anderen rheologischen Größen und die Sicherheitswerte blieben unbeeinflußt. Zusammenfassend ist festzuhalten, daß bereits 1 g Bärlauch die Mikrozirkulation und die Plasmaviskosität verbessern.

## Patentansprüche

1. Verwendung von Allium ursinum L. (Bärlauch) zur Herstellung eines Medikaments zur Behandlung oder Prophylaxe von Durchblutungsstörungen.

2. Verwendung nach Anspruch 1, **dadurch gekennzeichnet**, daß der Bärlauch in oraler Form verabreicht wird.

## Claims

1. Use of Allium ursinum L. (wild garlic) for the preparation of a drug for the treatment or prophylaxis of circulatory disorders.

2. Use according to Claim 1, **characterized in that** the wild garlic is administered in oral form.

## Revendications

1. Utilisation d'allium ursinum L (ail d'ours) pour la préparation d'un médicament en vue du traitement ou de la prophylaxie de troubles circulatoires.

2. Utilisation suivant la revendication 1, caractérisée en que l'ail d'ours est administré par voie orale.
